(19) Europäisches Patentamt
European Patent Office
Office européen des brevets

(11) Veröffentlichungsnummer: **0 408 928 A1**

(12) # EUROPÄISCHE PATENTANMELDUNG

(21) Anmeldenummer: **90111941.2**

(51) Int. Cl.5: **C07C 37/14**, C07C 39/15

(22) Anmeldetag: **23.06.90**

(30) Priorität: **08.07.89 DE 3922518**

(43) Veröffentlichungstag der Anmeldung:
**23.01.91 Patentblatt 91/04**

(84) Benannte Vertragsstaaten:
**CH DE ES FR GB IT LI NL**

(71) Anmelder: **BAYER AG**

**D-5090 Leverkusen 1 Bayerwerk(DE)**

(72) Erfinder: **Arndt, Uwe, Dr.**
**Malteserstrasse 85**
**D-5000 Koeln 40(DE)**
Erfinder: **Block, Hans-Dieter, Dr.**
**Biesenbach 49**
**D-5090 Leverkusen 3(DE)**
Erfinder: **Fuhr, Karl, Dr.**
**Krüllsdyk 55**
**D-4150 Krefeld(DE)**
Erfinder: **Müller, Friedemann, Dr.**
**Am Steinacker 5**
**D-4040 Neuss 21(DE)**

(54) **Verfahren zur Herstellung von alpha-Methylbenzyl-substituierten Phenolen.**

(57) Die vorliegende Erfindung betrifft ein Verfahren zur Herstellung von α-Methylbenzylphenolen bzw. von Gemischen dieser α-Methylbenzylphenole und unsubstituierter Phenole durch Umsetzung von gegebenenfalls mit $C_1$- bis $C_4$-Alkylgruppen substituierten Phenolen oder eines Phenols mit Styrol, wobei die Umsetzung von gegebenenfalls mit $C_1$- bis $C_4$-Alkylgruppen substituierten Phenolen oder eines Phenols mit Styrol durch Zusatz von 0,01 bis 100 Mol-% eines Phosphorchorids - bezogen aufd das Phenol - bei Temperaturen von 20 bis 350° C erfolgt.

EP 0 408 928 A1

## VERFAHREN ZUR HERSTELLUNG VON α-METHYLBENZYL-SUBSTITUIERTEN PHENOLEN

Die Synthese der substituierten oder unsubstituierten Mono-, Di-, Tri-α-methylbenzylphenole kann unter den bei der Kernalkylierung von Phenolen üblichen Bedingungen erfolgen. Als Katalysatoren eignen sich hierbei insbesondere Schwefelsäure, Phosphorsäure und Sulfonsäuren sowie saure Kationenaustauscher und die allgemein bekannten typischen Friedel-Crafts-Katalysatoren wie z.B. Aluminiumchlorid, Bortrifluorid und Eisen(III)-chlorid oder Mischungen dieser Katalysatoren mit Säuren wie z.B. Borsäure oder Schwefelsäure.

Die Verwendung von Mineralsäuren und der anderen bekannten und genannten Katalysatoren bei der Kernalkylierung ist im allgemeinen mit dem Nachteil verbunden, daß der saure Katalysator nach der Reaktion durch Neutralisation und/oder durch Auswaschen aus dem Endprodukt entfernt werden muß. Besonders das Auswaschen führt neben Phenol-Verlusten bei der technischen Anwendung zu erheblichen Abwasser- und Korrosionsproblemen. Um diese Nachteile zu umgehen, wurde schon mehrfach vorgeschlagen, unlösliche, stark saure Katalysatoren zu verwenden. Unter diesen Festsäuren haben insbesondere saure Ionenaustauscherharze wie Polystyrolsulfonsäure-Harze oder sulfonierte Phenol-Formaldehyd-Harze als Katalysatoren für Kernalkylierungen von Phenolen Bedeutung erlangt, da sie sich nach abgeschlossener Reaktion leicht aus dem Endprodukt durch Filtration beseitigen lassen. Nachteilig erweisen sich bei dieser Vorgehensweise allerdings die längeren Reaktionszeiten, die höheren Temperaturen sowie ein notwendiger größerer Phenol-Überschuß und die beim Arbeiten mit gemischt fest-flüssigen Systemen allgemein auftretenden Probleme.

Aufgaben der vorliegenden Erfindung war es daher, ein Verfahren zur Synthese von α-Methylbenzyl-substituierten Phenolen zu entwickeln, das die oben genannten Nachteile nicht aufweist und das im Stande ist, die zur Herstellung von α-Methylbenzylphenylestern Phosphor enthaltender Säuren, das sind insbesondere Phosphorsäure und Phosphorige Säure, Phosphonsäuren, Phosphonige Säuren, Phosphinsäuren und Phosphinige Säuren sowie die Thioderivate der Säuren mit fünfwertigem Phosphor, als Zwischenprodukt benötigten Phenole ohne notwendige Zwischenreinigung bzw. Entfernung des eingesetzten Katalysators zu liefern.

Erfindungsgemäß wird diese Aufgabe durch ein Verfahren zur Herstellung von α-Methylbenzylphenolen der allgemeinen Formel

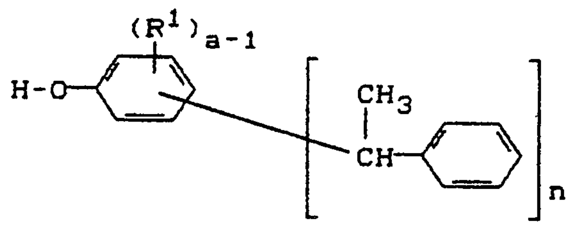

worin a und n unabhängig voneinander eine ganze Zahl von 1 bis 3, $R^1$ eine Alkylgruppe mit 1 bis 4 Kohlenstoff-Atomem bedeutet, bzw. von Gemischen dieser α-Methylbenzylphenole und unsubstituierter Phenole gelöst, welches dadurch gekennzeichnet ist, daß die Umsetzung von gegebenenfalls mit $C_1$ bis $C_4$ Alkylgruppen substituierten Phenolen oder eines Phenols mit Styrol durch Zusatz eines Phosphorchlorides bei Temperaturen von 20 bis 350 °C erfolgt.

Für das Verfahren der Erfindung geeignete Phenole sind z.B. Phenol, die isomeren Kresole, die isomeren Xylenole die isomeren Mono-, und Diethylphenole, die isomeren Mono- und Diisopropylphenole, die isomeren Mono-und Di- tert. butylphenole, i-Oktylphenol, Nonylphenol, Phenylphenol, Isopropylphenylphenole, Dodecylphenol, Methylenphenylphenol, Isopropylidenphenylphenol, Cyclohexylphenol als Einzelsubstanzen, als Gemisch sowie gegebenenfalls im Gemisch mit weiteren z.B. dreifach substituierten Phenolen.

Von den für das erfindungsgemäße Verfahren geeigneten Phosphorchloriden seien beispielhaft genannt: Phosphoroxychlorid, Phosphortrichlorid, Phenyldichlorphosphat, Diphenylchlorphosphat, Kresylphenylchlorphosphat, Phenyldichlorphosphit, Methanphosphonsäuredichlorid, Ethanphosphonsäuredichlorid, Vinylphosphonsäuredichlorid, Chlormethanphosphonsäuredichlorid, Benzolphosphonsäuredichlorid, Methylethylchlorphosphan, Diphenylchlorphosphan, Methylethylphosphinsäurechlorid, Diphenylphosphinsäurechlorid, Methyldichlorphosphan, Phenyldichlorphosphan, 1-Chlor-1-oxophospholin, Methanphosphonsäurephenylesterchlorid, Benzolphosphonsäurekresylesterchlorid.

Besonders bevorzugt ist ein Verfahren zur Herstellung von α-Methylbenzylphenolen, welches dadurch gekennzeichnet ist, daß als Phosphorchloride Phosphoroxychlorid oder Phosphortrichlorid eingesetzt werden.

Weiterhin bevorzugt ist ein Verfahren zur Herstellung von α-Methylbenzylphenolen, welches dadurch gekennzeichnet ist, daß als Phosphorchlorid ein organisches Phosphorchlorid der Formel

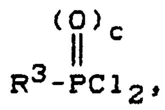

worin $R^3$ ein aliphatischer oder aromatischer Rest und c 0 oder 1 ist, eingesetzt wird.

Das erfindungsgemäße Verfahren wird in einer allgemeinen Form wie folgt ausgeführt:

Das zu substituierende Phenol wird mit Styrol in einem Molverhältnis von 0,01 bis 300 Mol-% Styrol, vorzugsweise 10-100 Mol-% Styrol, in Gegenwart von 0,01 bis 100 Mol-% eines als Katalysator eingesetzten Phosphorchlorides - bezogen auf Phenol - bei Temperaturen von 20 bis 350 °C in Kontakt gebracht.

Bei kleinen Ansätzen wird zu der entsprechend der gewünschten Zusammensetzung des Phenol-Endproduktes zusammengesetzten Mischung von Phenol und Styrol bei einer eine zügige Umsetzung ermöglichenden Temperatur, normalerweise einer erhöhten Temperatur, das Phosphorchlorid üblicherweise unter Rühren mit einer solchen Geschwindigkeit zugeführt, daß die gewünschte Reaktionstemperatur gehalten werden kann. Bei größeren Ansätzen, bei denen die Abführung der freiwerdenden Reaktionswärme Schwierigkeiten bereiten kann, empfiehlt es sich, das Phenol und das Phosphorchlorid vorzulegen und die Styrolmenge sukzessive nachzuführen.

Nachdem die Reaktion beendet ist, wird entsprechend den Materialeigenschaften und den Anforderungen an das Endprodukt, z.B. durch Abziehen flüchtiger Anteile im Vakuum oder durch Behandeln mit Wasser oder wäßrigen Lösungen, das Phenol-Gemisch gereinigt. Bei Verwendung der benzylierten Phenole für die Synthese phosphorenthaltender Säureester kann das Phenol-Gemisch ohne weitere Reinigung eingesetzt werden.

In einer bevorzugten Ausführungsform wird das erfindungsgemäße Verfahren in folgender Weise durchgeführt:

Die entsprechend der gewünschten Produktzusammensetzung erforderlichen Mengen Phenol und Styrol, und das als Katalysator erforderliche Phosphorchlorid werden kontinuierlich, gegebenenfalls unter Rühren und Erhitzen, in eine mehrstufige Reaktionskaskade eindosiert. Nach erfolgter vollständiger Benzylierung wird das ablaufende Rohprodukt, sofern erforderlich, entsprechend den durch die Anwendung vorgegebenen Anforderungen durch kombinierte Destillation und Wäsche gereinigt, andernfalls wird es ohne weitere Reinigung mit weiterem Phosphorchlorid derselben Art wie oben eingesetzt zu Estern der zugehörigen Phosphor enthaltenden Säure umgesetzt, wobei zusätzliche, nicht Methylbenzyl-Gruppen enthaltende Phenole von Beginn an zugesetzt oder später nachgeführt werden können.

Die nach dem erfindungsgemäßen Verfahren hergestellten Produkte werden insbesondere als Edukte bei der Synthese benzylphenylsubstituierter Phosphorester, die ihrerseits Anwendungen als Hydraulikflüssigkeiten, Weichmacher und Flammschutzmittel finden, eingesetzt.

Das erfindungsgemäße Verfahren soll anhand der folgenden Beispiele noch näher erläutert werden (Gew.-Angaben sind - soweit nicht anders vermerkt ist - Gew.-%).

## Beispiele

### Beispiel 1

In einem Reaktionsgefäß wird eine Mischung aus 1 Mol Kresol, 0,05 Mol Phosphoroxychlorid und 1 Mol Styrol auf 130-150 °C erhitzt. Die anschließend stark exotherm einsetzende Reaktion verläuft innerhalb von 30 Minuten quantitativ. Die Reaktion liefert ein Phenol-Rohprodukt, das neben 12 % unsubstituiertem Kresol 88 % mono- bzw. mehrfach benzylierte Kresole enthält.

Beispiel 2

In einem Reaktionsgefäß werden 1 Mol Hydrochinon mit 0,05 Mol Phosphoroxychlorid angeschmolzen. Bei 175 °C wird 1 Mol Styrol langsam zugetropft, wodurch die Temperatur bis auf 210 °C ansteigt. Nach vollständiger Zugabe und abgeschlossener Addition wird ein Phenol-Rohprodukt erhalten, bei den neben 4 %. unsubstituiertem Hydrochinon 96 % mono- bzw. mehrfach benzylierte Hydrochinone vorliegen.

Beispiel 3

Zu einer Mischung aus 0,5 Mol Nonylphenol und 0,05 Mol Phosphoroxychlorid wird bei 150 °C 0,5 Mol Styrol zugetropft. Die stark exotherm verlaufende Reaktion liefert ein Rohprodukt, das neben 6 % unsubstituiertem Nonylphenol 94 % mono- bzw. mehrfach benzylierte Nonylphenole enthält.

Beispiel 4

Zu einer Mischung aus 0,5 Mol 4-Hydroxy-biphenyl und 0,06 Mol Phosphortrichlorid wird bei 150 °C 0,5 Mol Styrol zugetropft. Im Verlauf der Reaktion wird die Temperatur auf 200 °C erhöht. Nach vollständiger Zugabe wird noch zwecks Vervollständigung der Addition eine halbe Stunde nachgerührt. Es wird ein Reaktionsrohprodukt erhalten, das neben ca.15 % unsubstituiertem 4-Hydroxybiphenyl 85 % mono- bzw. mehrfach benzylierte Additionsprodukte enthält.

Beispiel 5

Zu einer Mischung aus 0,5 Mol Cyclo-hexylphenol und 0,06 Mol $PCl_3$ wird bei 150 °C langsam 0,5 Mol Styrol zugetropft. Nach erfolgter Zugabe wird die Temperatur zur Vervollständigung der Addition noch 30 min auf 180 bis 200 °C erhöht. Beim Abkühlen kristallisiert ein Reaktionsprodukt, in dem neben ca. 25 % unsubstituiertem Cylo-Hexylphenol 75 % mono- bzw. mehrfach benzylierte Produkte vorliegen.

Beispiel 6

In eine dreistufige Reaktionskaskade mit je 250 ml Verweilvolumen/Stufe werden kontinuierlich 216 g/h Kresol, 208 g/h Styrol und 17 g/h Phosphoroxychlorid eindosiert. Die Temperatur wird über die einzelnen Verweilstufen bis auf 180 bis 190 °C gesteigert. Aus dem letztem Verweilgefäß läuft ein Rohprodukt ab, das neben 15 % Kresol 85 % mono- bzw. mehrfach benzylierte Kresole enthält.

Beispiel 7

In eine dreistufige Reaktionskaskade mit je 250 ml Verweilzeit Vol./Stufe werden kontinuierlich 500 ml/h eines Gemisches aus 47,5 % Phenol und 52,5 % Styrol sowie 12 ml/h Phosphoroxychlorid eindosiert. Die Temperatur wird über die einzelnen Verweilstufen bis auf 180 °C gesteigert. Das entstandene Reaktionsprodukt weist eine Zusammensetzung von ca. 5 % Phenol und 95 % mono-bzw. mehrfach benzylierter Phenole auf.

Beispiel 8

Zu einer Mischung aus 1,0 Mol Phenol und 0,05 Mol $POCl_3$ wird bei 130 bis 140 °C langsam 0,2 Mol Styrol zugetropft. Nach vollständiger Zugabe und Abklingen der exothermen Reaktion wird ein Phenol-Rohprodukt erhalten, daß neben 66 %. unsubstituiertem Phenol 34 % mono- bzw. mehrfach benzylsubstituierte Phenole enthält.

Beispiel 9

In ein Reaktionsgefäß werden zu einer Mischung aus 1,0 Mol Phenol und 0,1 Mol Methanphosphonsäuredichlorid bei 120 bis 130 °C langsam unter Rühren 1,0 Mol Styrol zugetropft. Nach vollständiger Zugabe wird das Reaktionsgemisch noch 30 min. bei 200 °C nachgerührt. Die Reaktion liefert ein Phenol Rohprodukt , das neben 15 % unsubtituiertem Phenol 85 % mono- bzw. mehrfach benzylierte Phenole enthält.

## Ansprüche

1. Verfahren zur Herstellung von α-Methylbenzylphenolen der allgemeinen Formel

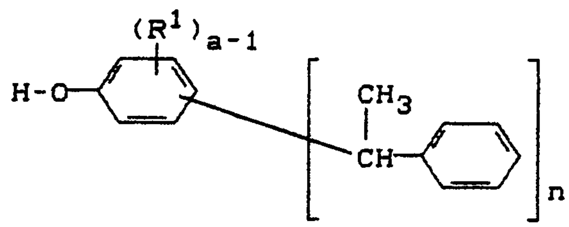

worin a und n unabhängig voneinander eine ganze Zahl von 1 bis 3, $R^1$ eine Alkylgruppe mit 1 bis 4 Kohlenstoff-Atomem bedeutet, bzw. von Gemischen dieser α-Methylbenzylphenole und unsubstituierter Phenole durch Umsetzung von gegebenenfalls mit $C_1$ bis $C_4$-Alkylgruppen substituierten Phenolen oder eines Phenols mit Styrol dadurch gekennzeichnet, daß die Umsetzung von gegebenenfalls mit $C_1$ bis $C_4$-Alkylgruppen substituierten Phenolen oder eines Phenols mit Styrol durch Zusatz von 0,01 bis 100 Mol % eines Phosphorchlorides - bezogen auf das Phenol - bei Temperaturen von 20 bis 350 °C erfolgt.

2. Verfahren nach Anspruch 1, dadurch gekennzeichnet, daß als Phosphorchlorid Phosphoroxychlorid oder Phosphortrichlorid eingesetzt wird.

3. Verfahren nach Anspruch 1, dadurch gekennzeichnet, daß das Phosphorchlorid ein organisches Phosphorchlorid der Formel

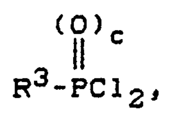

worin $R^3$ ein aliphatischer oder aromatischer Rest und c 0 oder 1 ist, eingesetzt wird.

4. Verfahren nach einem der Ansprüche 1 bis 3, dadurch gekennzeichnet, daß die Umsetzungen in einem kontinuierlichen Prozeß ausgeführt werden.

5. Verfahren nach einem der Ansprüche 1 bis 4, dadurch gekennzeichnet, daß als Phenol nur das unsubstituierte Phenol eingesetzt wird.

6. Verfahren nach einem der Ansprüche 1 bis 5, dadurch gekennzeichnet, daß das für die Umsetzung zu α-Methylbenzyl-substituierten Phenolen bzw. Phenylestern benötigte Styrol in unterstöchiometrischer Menge eingesetzt wird und Gemische von α-Methylbenzyl-substituierten Phenolen mit α-Methylbenzylunsubstituierten Phenolen hergestellt werden.

7. Verfahren nach einem der Ansprüche 1 bis 6, dadurch gekennzeichnet, daß als Katalysator ein Gemisch verschiedener Phosphorchloride eingesetzt wird.

8. Verwendung der nach einem der Ansprüche 1 - 7 hergestellten α-Methylbenzylphenole zur Herstellung von Estern Phosphor enthaltender Säuren.

Europäisches Patentamt

# EUROPÄISCHER RECHERCHENBERICHT

Nummer der Anmeldung

**EP 90 11 1941**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WORLD PATENT INDEX DATABASE Sektion E14, Woche 38, 1970, Derwent Publications Ltd., London, GB; & SU-A-255288 (TOPCHIEV PETROSYNTHESIS) | 1,2,5 | C 07 C 37/14<br>C 07 C 39/15 |
| Y | FR-A-1 111 248 (DISTILLERS)<br>* Seite 2, Spalte 1, Zeilen 20-22 * | 1,2,5 | |
| Y | EP-A-0 049 126 (COALITE)<br>* Ansprüche 5,6 * | 1,2,5 | |
| P,A | EP-A-0 335 674 (YOSHITOMI PHARMACEUTICAL INDUSTRIES)<br>* Seite 4, Zeilen 54-56 * | 1 | |
| A | US-A-2 655 547 (BRYNER)<br>* Spalte 1, Zeilen 38-42 * | 1 | |
| A | US-A-3 415 907 (SCONCE)<br>* Spalte 1, Zeile 70 * | 8 | |

RECHERCHIERTE SACHGEBIETE (Int. Cl.5)

C 07 C 37/00
C 07 C 39/00

Der vorliegende Recherchenbericht wurde für alle Patentansprüche erstellt

| Recherchenort | Abschlußdatum der Recherche | Prüfer |
|---|---|---|
| Berlin | 16 Oktober 90 | PROBERT C.L. |

KATEGORIE DER GENANNTEN DOKUMENTE
X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument